# EUROPEAN PATENT APPLICATION

(11) **EP 1 685 822 A1**
(43) Date of publication of application: **02.08.2006**
(21) Application number: 04799424.9
(22) Date of filing: 01.11.2004

(54) **GLUCOSE ABSORPTION INHIBITOR AND PROCESS FOR PRODUCING THE SAME**

(30) Priority: 05.11.2003 JP 2003375323
(71) Applicant: Nichirei Foods Inc., Tokyo 104-8402 (JP)
(72) Inventor: AOKI, Hitoshi Nichirei Corp., Research & Dev. Div., Chiba-shi Chiba 261-8545 (JP); HANAMURA, T. Nichirei Corp., Research & Dev. Div., Chiba-shi Chiba 2618545 (JP); MAYAMA, C. Nichirei Corp., Research & Dev. Div., Chi-shi Chiba 2618545 (JP); HIRAYAMA, Yasushi, Foods Safety Research Center, Mihaama-ku, Chiba-shi2618545 (JP); SHIMIZU, Makoto, The University of Tokyo, Bunkyo- ku Tokyo 1138657 (JP)
(74) Representative: Woods, Geoffrey Corlett
(86) International application number: PCT/JP2004/016218
(87) International publication number: WO 2005/044290

(57) **Abstract**

This invention provides an inhibitor of glucose absorption and a method for producing the same. Such inhibitor of glucose absorption comprises, as an active ingredient, an acerola-derived substance capable of inhibiting glucose absorption.

## Description

### Technical Field

The present invention relates to an inhibitor of glucose absorption comprising, as an active ingredient, an acerola-derived substance capable of inhibiting glucose absorption.

### Background Art

In accordance with changes in dietary habits and lifestyle of recent years, the number of diabetic patients is increasing. At present, the number of diabetic patients is as high as 7,000,000 in Japan, and such number could be as large as 15,000,000 when future diabetics are added.

Diabetes is a metabolic disorder in which a prolonged hyperglycemic state caused by an insufficient level of insulin hormone activities is exhibited. A prolonged hyperglycemic state may result in development of various types of complications, such as nervous disorders, cataract, renal disorders, retinopathy, arthrosclerosis, atherosclerosis, and diabetic gangrene.

Diabetes is primarily treated via diet therapy. Since severe dietary restrictions are required, diet therapy imposes serious daily life and psychological stresses on patients. For example, saccharides in boiled rice or bread are broken down into glucose in the intestinal tract and absorbed by the body. The overconsumption of glucose requires insulin and irritates the pancreas. Accordingly, the overconsumption of glucose is not preferable for patients during diet therapy.

If glucose absorption in the intestinal tract could be inhibited, the appetite could be satisfied while avoiding the overconsumption of saccharides.

Accordingly, several inhibitors of glucose absorption were developed in the past, although many of them may cause side effects. Therefore, the development of preparations that are harmless to humans is strongly desired.

Under the above circumstances, inhibitors of glucose absorption made from natural ingredients that are considered to be less likely to cause side effects, such as an inhibitor of glucose absorption comprising, as an active ingredient, epicatechin gallate (JP Patent Publication (Unexamined) No. 11-302168 (1999)), roasted tea of Gymnema inodorum capable of inhibiting glucose absorption (European Patent Application No. 0861595), and leaf extracts of Gymnema inodorum capable of inhibiting glucose absorption (JP Patent Publication (Unexamined) No. 8-149965 (1996)) have been produced. However, the development of such inhibitors is still insufficient.

Polyphenols have antioxidative effects and thus have drawn attention as natural ingredients that are effective in preventing so-called lifestyle-related diseases, such as arteriosclerosis, diabetes, and cancer.

Acerola is a tropical fruit of the genus *Malpighia* of the family *Malpighiaceae*, which is native to Caribbean Islands. It is known that acerola fruit contains abundant vitamin C at levels of approximately 1,500 mg or more per 100 g thereof. It recently has been used for beverages and health food products throughout the world. Because of its bright red color, it is expected that acerola contains a wide variety of polyphenol components and that it could be used for a wide variety of applications.

### Disclosure of the Invention

An object of the present invention is to provide an inhibitor of glucose absorption comprising, as an active ingredient, an acerola-derived substance capable of inhibiting glucose absorption.

It is another object of the present invention to provide an acerola-derived preventive and/or therapeutic agent for diabetes or diabetic complications.

The present inventors have conducted concentrated studies in order to attain the above objects. As a result, they have discovered that an acerola-derived substance is capable of inhibiting glucose absorption.

The present invention includes the following inventions.
(1) An inhibitor of glucose absorption comprising, as an active ingredient, an acerola-derived substance capable of inhibiting glucose absorption.
(2) An inhibitor of glucose absorption comprising, as an active ingredient, an acerola extract or a processed product thereof.
(3) An inhibitor of glucose absorption comprising, as an active ingredient, an acerola-derived polyphenol.
(4) The inhibitor of glucose absorption according to (3), wherein the polyphenol contains an anthocyanin pigment.
(5) The inhibitor of glucose absorption according to any of (1) to (4), which is used for treating diabetes and/or diabetic complications.
(6) A preventive and/or therapeutic agent for diabetes or diabetic complications comprising, as an active ingredient, an acerola-derived substance capable of inhibiting glucose absorption. The term "preventive and/or therapeutic agent for diabetes or diabetic complications" used herein refers to an agent that can be used for the prevention and/or treatment of diabetes or diabetic complications.
(7) A method for producing an inhibitor of glucose absorption comprising steps of: grinding acerola fruit; isolating an extract from the ground acerola fruit; and purifying the extract according to need.

This description includes part or all of the contents as disclosed in the description and/or drawings of Japanese Patent Application No. 2003-375323, which is a priority document of the present application.

The inhibitor of glucose absorption according to the present invention inhibits glucose absorption in the intestinal tract. Thus, the appetite can be satisfied while avoiding the overconsumption of saccharides. The inhibitor of glucose absorption according to the present invention is accordingly effective in the prevention and/or treatment of conditions or diseases in which the overconsumption of saccharides is not preferable (i.e., diabetes or diabetic complications, and more specifically, diabetic complications).

The active ingredient of the inhibitor of glucose absorption according to the present invention is a naturally occurring substance derived from acerola. Accordingly, such an inhibitor involves few side effects.

### Brief Description of the Drawings

Fig. 1 shows the effects of acerola-derived polyphenols on the inhibition of glucose uptake in the Caco-2 cell.
Fig. 2 shows the effects of acerola-derived polyphenols on the inhibition of 3-O-methyl glucose uptake in the Caco-2 cell.
Fig. 3 shows the concentration-dependent effects of a C18-adsorbed fraction of acerola on the inhibition of 3-O-methyl glucose uptake in the Caco-2 cell.
Fig. 4 shows the results of a comparison of changes in blood glucose levels after the glucose administration with the elapse of time between the group to which the C18-adsorbed fraction of acerola had been administered and the control group.
Fig. 5 shows the results of a comparison of the area under the blood glucose curve between the group to which the C18-adsorbed fraction of acerola had been administered and the control group.

### Preferred Embodiments of the Invention

The inhibitor of glucose absorption according to the present invention may comprise, as an active ingredient, an acerola-derived substance capable of inhibiting glucose absorption. The area of production or the variety of acerola is not particularly limited. For example, acerola can be produced in Okinawa, Japan, or in Brazil.

Fruit juice, crushed or ground fruit, or powdered fruit of acerola may be used. An acerola extract or a processed product thereof can also be used. The term "fruit" used herein refers to the whole fruit, including edible parts and seeds.

An acerola extract or a processed product thereof can be obtained from acerola fruit with the use of water (e.g., pure water or purified water), an organic solvent, or the like. A hydrophilic organic solvent is particularly preferable, and such solvent can be selected from among conventional organic solvents. Examples include alcohols, such as methyl alcohol, ethyl alcohol, glycerin, propylene glycol, and 1,3-butylene glycol, acetone, tetrahydrofuran, acetonitrile, 1,4-dioxane, pyridine, dimethyl sulfoxide, N,N-dimethylformamide, and acetic acid. Use of methyl alcohol is particularly preferable. Such hydrophilic organic solvents, particularly methyl alcohol and ethyl alcohol, are preferably mixed with water.

The conditions for extraction are not particularly limited. The amount of an extraction solvent used is generally about 100 to 1,000 parts by weight, and preferably about 200 to 500 parts by weight, based on 100 parts by weight of fruit. The temperature range is generally between 0°C and 120°C, and preferably between 20°C and 50°C, from the viewpoint of extraction efficiency. The duration of extraction is about 1 to 24 hours, and preferably for about 1 or 2 hours.

The residue remaining after the extraction may be removed via filtration or centrifugation. Also, an extract can be further concentrated to the desired level via vacuum concentration or other means according to need. Further, the extract may be dehydrated via lyophillization, spray drying, or other means to form powders. In such a case, the extract may be mixed with an excipient or the like for dehydration.

The resulting extract contains abundant saccharides or organic acids. Accordingly, it is preferable to carry out a step of purification in order to remove such substances. Purification may be carried out via, for example, normal-phase or reverse-phase chromatography, ion-exchange chromatography, or gel filtration. These techniques may be carried out in combination.

The extract was isolated and purified. This revealed that an acerola-derived polyphenol component contains anthocyanin pigments, such as cyanidin-3-rhamnoside and pelargonidin-3-rhamnoside. It is known that anthocyanin pigments have antioxidative effects. In addition to such effects, the present inventors discovered that an acerola-derived anthocyanin pigment has the capacity to inhibit glucose absorption.

An extract can be isolated and purified via, for example, HPLC, chromatography using a synthetic absorbent, ion-exchange chromatography, or gel filtration. Chromatography using a synthetic absorbent is particularly preferable. In such a case, the extract is preferably eluted with a 10% to 100% ethanol solution, for example.

The inhibitor of glucose absorption according to the present invention can be in the form of a preparation comprising an acerola extract or a purified product thereof in combination with a conventional pharmaceutical carrier. Dosage form is not particularly limited, and it is adequately determined according to need. In general, dosage forms can be oral preparations, such as tablets, capsules, granules, fine granules, powders, pills, liquids, syrups, suspensions, emulsions, or elixirs or parenteral preparations, such as injections, drops, suppositories, inhalants, transdermal absorbents, transmucosal absorbents, transnasal preparations, enteral preparations, adhesive preparations, or ointments. These preparations are used alone or in combinations of two or more in accordance with symptoms. The amount of the inhibitor of glucose absorption according to the present invention to be added varies depending on various conditions, such as the type of the substance to which the inhibitor is to be added or the type of usage. In general, it is preferable to add the inhibitor in amounts of 0.01 % to 20% by mass based on the total amount of the substance with which mixing takes place.

The dose of the inhibitor of glucose absorption according to the present invention varies depending on the age and the body weight of the patient, the severity of disease, and the route of administration. In the case of oral administration, dry powders of an acerola extract is usually administered in an amount of 10 mg to 3,000 mg per day, and the number of doses is usually 1 to 3 times per day.

Oral preparations are produced in accordance with a conventional technique using excipients, such as starch, lactose, sucrose, mannite, carboxymethylcellulose, cornstarch, or inorganic salt.

In addition to such excipients, this type of preparation can comprise, for example, a binder, a disintegrator, a surfactant, a lubricant, a flow promoter, a flavoring agent, a colorant, or a fragrant material.

Specific examples of binders include crystalline cellulose, crystalline cellulose carmellose sodium, methylcellulose, hydroxypropylcellulose, low-substituted hydroxypropylcellulose, hydroxypropylmethylcellulose, hydroxypropylmethylcellulose phthalate, hydroxypropylmethylcellulose acetate succinate, carmellose sodium, ethyl cellulose, carboxy methyl ethyl cellulose, hydroxyethyl cellulose, wheat starch, rice starch, cornstarch, potatostarch, dextrin, pregelatinized starch, partially pregelatinized starch, hydroxypropyl starch, Pullulan, polyvinylpyrrolidone, aminoalkyl methacrylate copolymer E, aminoalkyl methacrylate copolymer RS, mechacrylic acid copolymer L, mechacrylic acid copolymer, polyvinylacetal diethylaminoacetate, polyvinyl alcohol, gum Arabic, powdered acacia, agar, gelatin, white shellac, tragacanth, purified sucrose, and Macrogol.

Specific examples of disintegrators include crystalline cellulose, methylcellulose, low-substituted hydroxypropylcellulose, carmellose, carmellose calcium, carmellose sodium, croscarmellose sodium, wheat starch, rice starch, cornstarch, potatostarch, partially pregelatinized starch, hydroxypropyl starch, sodium carboxymethyl starch, and tragacanth.

Specific examples of surfactants include soybean lecithin, sucrose fatty acid ester, polyoxyl stearate, polyoxyethylene hydrogenated castor oil, polyoxyethylene polyoxypropylene glycol, sorbitan sesquioleate, sorbitan trioleate, sorbitan monostearate, sorbitan monopalmitate, sorbitan monolaurate, polysorbate, glyceryl monostearate, sodium lauryl sulfate, and lauromacrogol.

Specific examples of lubricants include wheat starch, rice starch, cornstarch, stearic acid, calcium stearate, magnesium stearate, hydrated silicon dioxide, light anhydrous silicic acid, synthetic aluminum silicate, dried aluminum hydroxide gel, talc, magnesium aluminometasilicate, calcium hydrogen phosphate, anhydrous dibasic calcium phosphate, sucrose fatty acid ester, waxes, hydrogenated vegetable oil, and polyethylene glycol.

Specific examples of flow promoters include hydrated silicon dioxide, light anhydrous silicic acid, dried aluminum hydroxide gel, synthetic aluminum silicate, and magnesium silicate.

When the inhibitor of glucose absorption of the present invention is administered in the form of a liquid, syrup, suspension, emulsion, or elixir, it may contain a taste and flavor corrigent or a colorant.

The inhibitor of glucose absorption of the present invention can be added to solid, semisolid, or liquid food products, or to food products in other forms, in accordance with conventional techniques. Examples of solid food products include, but are not limited to, block-shaped confectioneries such as biscuits and powdery food products such as powdered soup. Also, dry powders of an acerola extract can also be used as food products in such state. Examples of semisolid food products include capsules and jellies. Examples of beverages include fruit juice beverages, soft drink beverages, and alcoholic beverages. Alternatively, a beverage may be in the form of powder that is diluted with a liquid carrier such as water before ingestion. The inhibitor of glucose absorption according to the present invention can be added to food products or the like, and they can be prepared in the form of so-called foods for specified health uses (e.g., foods for preventing diabetes of its complications).

According to need, a stabilizer, a pH adjuster, saccharides, a sweetener, various vitamins, minerals, an antioxidant, an excipient, a solubilizer, a binder, a lubricant, a suspension, a moistening agent, a film-forming substance, a taste corrigent, a flavor corrigent, a colorant, a fragrant material, a preservative, and the like can be added to the aforementioned food products in accordance with conventional techniques.

Acerola, which is a starting material for the inhibitor of glucose absorption according to the present invention, has been heretofore applied to food products, beverages, cosmetics, and other applications, and its safety is assured.

### Examples

### Example 1: Preparation of C18-adsorbed fraction of acerola

Seeds were separated from acerola fruits, the remaining edible parts were homogenized, and 3x amount of methanol was added thereto, followed by 1-hour extraction. This procedure was carried out twice, and the extract was centrifuged, filtered, lyophilized, and then diluted in distilled water again. The resulting solution was applied to the C 18 cartridge columns (BAKERBOND spe®, C 18 disposable columns), washed with distilled water, eluted with a 0.2% TFA/methanol solution, and evaporated to dryness to obtain an extract. The resulting extract was designated as the C18-adsorbed fraction of acerola. The polyphenol content in the C18-adsorbed fraction of acerola was measured by the Folin-Denis method, and it was consequently found to be 22.7%.

### Example 2: Purification of acerola pigment component

Acerola juice was centrifuged, filtered, applied to the C18 cartridge columns (BAKERBOND spe®, C18 disposable columns), and washed with distilled water. Thereafter, the C18-adsorbed fraction was eluted with a 0.2% TFA/methanol solution and then subjected to separation and purification via HPLC. HPLC conditions were as follows.
Column: Inertsil ODS-2 columns (10.0 x 250 mm)
Column temperature: 40°C
Mobile phase A: 0.5% TFA/water solution
Mobile phase B: 0.5% TFA/acetonitrile solution
Gradient: linear gradient of mobile phase B from 0% to 100%
Flow rate: 2 ml/min
Detection: 520 nm

Through this procedure, 2 pigment components exhibiting absorption at 520 nm were observed. These 2 components were designated as pigment 1 and pigment 2. Pigment 1 and pigment 2 were further purified and subjected to NMR analysis. This revealed that pigment 1 was mainly composed of cyanidin-3-rhamnoside and that pigment 2 was mainly composed of pelargonidin-3-rhamnoside.

### Example 3: Test of glucose uptake by cultured Caco-2 cells derived from human intestinal tract

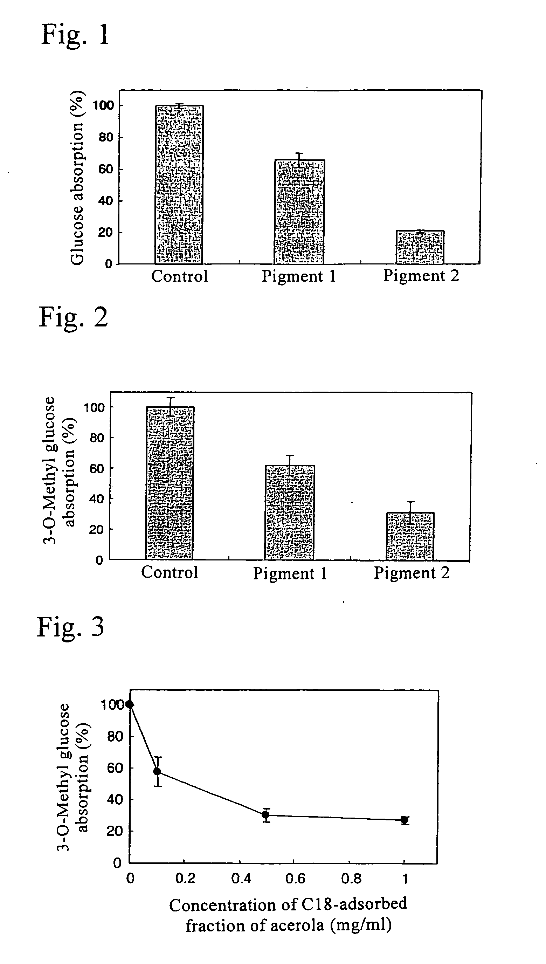

Cultured Caco-2 cells derived from human intestinal tracts were monolayer-cultured on a plate for about 10 days and then differentiated. A DMEM medium containing 10% fetal calf serum was used. Pigment 1 and pigment 2 (250 µg/ml each) were added to the differentiated Caco-2 cells, the resultants were preincubated for 15 minutes, and ³H-labeled glucose or 3-O-methyl glucose was then added thereto, followed by incubation for 10 minutes. ³H incorporated into the cells was analyzed to determine the amount of glucose or methyl glucose transported. The results are shown in Fig. 1 and in Fig. 2 as relative values (%) in relation to the controls. As is apparent from these figures, pigment 1 and pigment 2 mainly composed of the acerola-derived polyphenol specifically inhibited the glucose and 3-O-methyl glucose transportation. Subsequently, the C18-adsorbed fraction of acerola (up to 1 mg/ml) was added to the Caco-2 cells, and the amount of 3-O-methyl glucose transported was determined in the manner described above. This revealed that the C18-adsorbed fraction of acerola containing pigment 1 and pigment 2 inhibited the 3-O-methyl glucose transportation in a concentration-dependent manner (Fig. 3).

### Example 4: Glucose tolerance test on normal mice

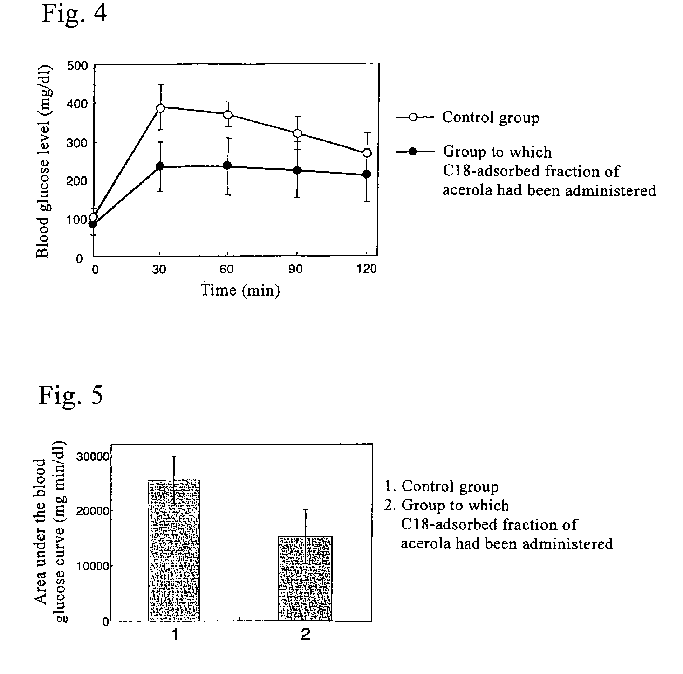

7-week-old ICR male mice were divided into 2 groups, i.e., the group of 5 mice to which the C18-adsorbed fraction of acerola had been administered and the control group of 5 mice. They were subjected to the glucose tolerance test. Mice (body weight: about 35 g) were made to fast overnight. The C18-adsorbed fraction of acerola was lysed with physiological saline, and 250 mg/kg (body weight) of the resultant was administered to the mice through an intragastric sonde. Physiological saline was administered to the control group. Glucose (2 g/kg (body weight)) was administered 30 minutes thereafter, blood was sampled from caudal veins every 30 minutes until 2 hours after the administration, and the blood glucose levels were assayed. The blood glucose levels were assayed using the Glucose CII-Test Wako (Wako Pure Chemical Industries). This revealed that an increase in the blood glucose level was suppressed in the group to which the C18-adsorbed fraction of acerola had been administered (Fig. 4) and that the area under the blood glucose curve was significantly reduced compared with the control group (Fig. 5).

All publications, patents, and patent applications cited herein are incorporated herein by reference in their entirety.

### Industrial Applicability

The inhibitor of glucose absorption according to the present invention inhibits glucose absorption in the intestinal tract. Thus, the appetite can be satisfied while avoiding the overconsumption of saccharides. The inhibitor of glucose absorption according to the present invention is accordingly effective in the prevention and/or treatment of conditions or diseases in which the overconsumption of saccharides is not preferable (i.e., diabetes or diabetic complications, and more specifically, diabetic complications).

## Claims

1. An inhibitor of glucose absorption comprising, as an active ingredient, an acerola-derived substance capable of inhibiting glucose absorption.

2. An inhibitor of glucose absorption comprising, as an active ingredient, an acerola extract or a processed product thereof.

3. An inhibitor of glucose absorption comprising, as an active ingredient, an acerola-derived polyphenol.

4. The inhibitor of glucose absorption according to claim 3, wherein the polyphenol contains an anthocyanin pigment.

5. The inhibitor of glucose absorption according to any of claims 1 to 4, which is used for treating diabetes and/or diabetic complications.

6. A preventive and/or therapeutic agent for diabetes or diabetic complications comprising, as an active ingredient, an acerola-derived substance capable of inhibiting glucose absorption.

7. A method for producing an inhibitor of glucose absorption comprising steps of: grinding acerola fruit; isolating an extract from the ground acerola fruit; and purifying the extract according to need.
